# EUROPÄISCHE PATENTSCHRIFT

(11) **EP 1 806 161 B1**
(45) Veröffentlichungstag und Bekanntmachung des Hinweises auf die Patenterteilung: **04.03.2009**
(21) Anmeldenummer: 06000430.6
(22) Anmeldetag: 10.01.2006
(51) Int. Cl.: A61N 5/10

(54) **Implantat zur Behandlung der Innenwände eines Resektionshohlraumes**
Implant for treatment of the inner walls of resection cavities
Implant pour le traitment des parois internes d'une cavité de résection

(43) Veröffentlichungstag der Anmeldung: 11.07.2007
(60) Teilanmeldung: 08015905.6
(73) Patentinhaber: Acrostak Corp. BVI, 8409 Winterthur (CH)
(72) Erfinder: Popowski, Youri, 1206 Genf (CH); Berger, Erwin, 9506 Stettfurt (CH)
(74) Vertreter: Weiss, Wolfgang

(56) Entgegenhaltungen:
- EP-A- 1 402 922
- EP-A- 1 568 397
- EP-A- 1 616 597
- WO-A-99/40962
- DE-A1- 19 808 170

## Beschreibung

Die Erfindung betrifft ein Implantat zur Behandlung der Innenwände eines Hohlraumes infolge einer Resektion.

Die Behandlung eines Gewebes innerhalb eines Hohlraumes infolge eines chirurgischen Eingriffs zur Entfernung eines Tumors hat in den vergangenen Jahren enorm an Bedeutung gewonnen. Der Gegenstand dieser Erfindung ist eine Einrichtung zur Strahlungstherapie.

In der EP 1402922 A1 ist ein derartiges Implantat offenbart. Dieses Implantat betrifft eine aufblasbare Kammer mit Vorrichtungen zur Einführung einer Strahlenquelle

Aus EP 16165597 A1 ist ein derartiges aus Modulen aufgebautem Implantat bekannt. Diese Schrift ist Stand der Technik gemäss Artikel 54(3) EPÜ und offenbart den Oberbegriff des Anspruchs 1.

Die US 4,815,449 offenbart eine Vorrichtung dieser Gattung, wobei das Implantat aus biologisch abbaubarem Material besteht.

Die Druckschrift US 6,673,006 offenbart eine Vorrichtung zur Anwendung einer Strahlentherapie, insbesondere einer Strahlentherapie möglichst nahe an dem zu bestrahlenden Medium (Brachytherapie).

Weiterer Stand der Technik ist aus den Druckschriften US 6,413,204, US 6,083,148, US 6,022,308, US 4,763,642 und US 5,913,813 bekannt.

Einige dieser im Stand der Technik offenbarten Einrichtungen sind zu gering dimensioniert, beispielsweise für intravasculäre Anwendungen. Andere Ausführungsformen bestehen aus einem Ballon mit zylindrischer Form, wobei der die Strahlungsquelle führende Katheter in Richtung der zentralen Achse verläuft.

Die Aufgabe der Erfindung besteht darin ein Implantat vorzuschlagen, welches auf eine Vielzahl von Anwendungsgebieten der Strahlungstherapie einsetzbar ist. Ferner soll das Implantat, welches in einen Resektionsraum eingeführt wird, elastisch sowohl in Längsrichtung als auch in radialer Richtung sein, in der Weise dass das Implantat nach einer Deformierung die ursprüngliche Form wieder einnehmen kann.

Darüber hinaus soll das Implantat die Möglichkeit liefern, die Strahlungsquelle mit variablem Abstand an die zu behandelnde Stelle zu bringen, d.h. die Dosis der Strahlung durch Veränderung des Abstandes zur inneren Wand des Resektionshohlraumes wählbar zu machen.

Eine weitere Aufgabe der Erfindung ist es, einige Bereiche, insbesondere die äusseren Dimensionen röntgenologisch zu erkennen.

Die Erfindung schlägt eine neue Vorrichtung zur Positionierung einer Strahlungsquelle zur Behandlung der inneren Wände eines Resektionshohlraumes vor. Zum Beispiel ist diese Vorrichtung anwendbar auf Resektionshohlräume in der Brust, Prostata, Gehirn oder andern zu behandelnden Resektionshohlräumen im menschlichen Körper, die infolge einer Entfernung eines Tumors entstanden sind.

Das Implantat soll eine solche eine elastische Flexibilität aufweisen, dass es sich der jeweiligen Geometrie des Resektionsraumes anpassen kann.

Erfindungsgemäss wird diese Aufgabe gelöst, durch die im Anspruch 1 definierten Merkmale.

Durch den modularen Aufbau des Implantates ergibt sich eine gewünschte optimale reversible Verformbarkeit. Sowohl die Module als auch ein aus mehreren Modulen aufgebautes System ist elastisch ausgebildet. Je nach Anwendung kann die Dimension in Längsrichtung des Implantates verändert und der Anwendung angepasst werden.

Vorzugsweise sind die Modulpaare kalottenförmig ausgebildet und miteinender verbindbar. Ebenso kann das Modul einstöckig und scheibenförmig mit nach aussen weisenden Wölbungen ausgebildet sein. Sowohl die aus in der Regel zwei Modulteile eines Modulpaares sind entweder steckbar miteinander verbindbar oder lose auf einen Führungskatheter aufgereiht. Das gleiche gilt für einstückig ausgebildete Module.

Gemäss einer besonders bevorzugten Ausführungsform erfolgt die Verbindung durch an den Modulteilen angeordnete Steckverbindungen. Ebenso kann die Verbindung durch separate Steckverbindungen erfolgen. An den Verbindungsstellen der Modulteile bzw. der Modulpaare sind die Verbindungen so beweglich, dass sich das aus mehreren Modulpaaren aufgebaute Implantat flexibel verhalten kann. Das Implantat weist elastische Flexibilität auf, d.h. das verformte Implantat kann in seine Ursprungsform zurückgehen.

Diese Elastizität kann durch konstruktive Elemente und/oder geeignete Materialien erzielt werden.

Die konstruktive Flexibilität wird durch federnde Elemente erzielt, die verschiedenartig geformt sein können, beispielsweise C, S-, Z- oder schraubenfederförmig. Die jeweiligen Formen weisen verschiedene Elastizitätskonstanten auf. Je nach Anwendung wird die geeignete Form eingesetzt.

Die materielle Elastizität wird durch die geeignete Wahl eines biologisch abbaubaren Materials erzielt, abhängig von der gewünschten Elastizität.

Im folgenden ist beispielhaft eine Liste von Materialien erwähnt. Diese Liste ist nicht als erschöpfend anzusehen. Alle verwandten und ähnliche Stoffe mit den erforderlichen Eigenschaften sind anwendbar:
Synthetische Polymere, Polymilchsäure, allgemein: auf Glycol- und Milchsäure basierende Polymere und Copolymere, Polycaprolactane, allgemein Polyhydroxylalkane (PHAs), Polyhydroxy alkanische Säuren = alle Polyester, Polyethylenglycol, Polyvinylglycol, Polyortoester, Polyanhydride, Polykarbonate, Polyamide, Polyimide, Polyimine, Polyiminokarbonate, Polyethylenimine, Polydioxane, Polyethylenoxide, Polyphosphazene, Polysulphone, Polyacrylsäuren, Polymethylmethanacrylate (PMMA), Polyacrylamide, Polyacrylnitrile, Polycyanoacrylate, Poly HEMA, Polyurethane, Polyolefine, Poystyrene, Polyteraphthalate, Polyfluoride, Polyethylene, Polypropylene, Polyetherketone, Polyvinylchloride, Silikone, Polysilikate (bioaktives Glas), Siloxane (Polydimethylsiloxane), Hydroxylapatite, natürliche Polymerderivate, z.B. Polyaminosäuren (natürliche und nicht-natürliche) möglich mit anderen Verbindungsblöcken wie Fettdikarbonsäuren und Diole, Polyester, Poly-betaaminoesther, allgemein: Polypeptide, wie Albumine, Alignate, Zellulose, zellulose Biozusammensetzungen, Zelluloseazetate, Chitin, Chitosan, Kollagene, Fibrine, Fibrinogene, Gelantine, Lignine, Stärkezusammensetzungen mit Miittlerem und hohem Anteil von Stärke, geschäumte Stärke, auf Soja basierende Kunststoffe, neutrale Polysaccharide (Gellan, Gummi, Pullulan, Lamarin und Kurdlan), auf Proteine basierende Polymere, wie Polylysin, Polyglutamate, Polymalonate, Polyhyaluronicsäuren, Polynucleinsäuren, Polysaccharide, Polyhydroxyalkane, Polyisoprene, auf Stärke basierende Polymere und alle Kopolymere, wie linear, schwach und stark verzweigt, die dazugehörigen Dendrite, kreuzweise verzweigt, mit funktionellen Eigenschaften, wie (geeignete Oberfläche, funktionale Gruppen, hydrophil oder hydrophob).

Die Steckverbindungen können auch als Muffen- Steckverbindung ausgebildet sein. Weiterhin ist es von Vorteil, dass die Steckverbindungen gegen Torsion gesichert sind.

Gemäss einer besonders bevorzugten Ausführungsform weisen die Modulteile, Module Durchführungen für die Katheter und/oder Führungen für die Strahlungsquellen auf. Die Durchführungen sind mittels federnder Elemente untereinander und/oder mit dem Umfang der Modulteile verbunden. Dies führt zu der gewünschten, wählbaren elastischen Flexibilität in radialer Richtung. Die zentralen Durchführungen können auch keine Steckverbindungen aufweisen. Es genügt z.B. wenn die einzelnen Elemente auf einem Führungskatheter aufgereiht sind. Die Wände des Resektionshohlraumes halten die einzelnen Elemente in ihrer gewollten Form zusammen.

Vorzugsweise bestehen die Implantate aus mindestens einem Modulpaar, wobei die Modulpaare eine flexible Kette bilden. Das Implantat kann beliebig lang durch Erhöhung der Anzahl der zusammengesteckten oder unverbundenen Modulpaare ausgestaltet sein. Durch die radial-flexibel gelagerten Durchführungen in den Modulteilen ist es in einfacher Weise möglich, die Katheter bzw. Führungen für die Strahlungsquellen durch ein kettenförmiges Implantat zu führen. Die Katheter bzw. Führungen für die Strahlungsquellen können nach der Behandlung aus dem Implantat herausgezogen werden, wobei das Implantat im Resektionsraum zurückbleibt.

Um das Implantat in den Körper einführen zu können wird es mit einem Führungskatheter versehen, der an einem Ende eine Nadel und an dem anderen Ende einen Stopper aufweist. Vorzugsweise ist die Nadel nach der Einführung des Implantates in den Körper entfernbar. Der Stopper ist verformbar, so dass der Führungskatheter nach Überwindung der Stoppwirkung störungsfrei und rückstandsfrei entfernbar ist. Weiterhin können mehrere Einzugskatheter gleichzeitig durch die in den Modulen dafür vorgesehene Durchführungen in das Implantat eingezogen werden, um beispielsweise die Strahlungsdosis zu erhöhen.

Von Bedeutung ist die Tatsache, dass die Zerfallzeit des biologisch abbaubaren Materials sich nicht wesentlich von der Zerfallszeit der chirurgischen Fäden für die Nähte des Resektionsraumes unterscheidet. Es soll damit verhindert werden, dass sich das Implantat in dem Resektionshohlraum nachteilig frei bewegen kann.

Ausführungsbeispiele sind in der Zeichnung dargestellt. Es zeigen:
- Fig. 1a und 1b: zwei zueinander passende Modulteile
- Fig. 2: ein Modulpaar
- Fig. 3: ein kettenförmiges Implantat
- Fig. 4: ein Querschnitt eines kettenförmigen Implantates
- Fig. 5: eine Draufsicht eines Modulteiles
- Fig. 6: eine dreidimensionale Ansicht eines Modulteiles
- Fig. 7: eine weiteres kettenförmiges Implantat
- Fig. 8: eine andere Ausführungsform eines Moduls in Draufsicht
- Fig. 9: die Ausführungsform von Figur 8 in Seitenansicht
- Fig. 10a und b: eine kettenförmiges Implantat mit Modulen nach Figur 8
- Fig. 11a und b: eine schematische Anordnung eines Implantates
- Fig. 12: ein Implantat mit Führungskatheter im Körper
- Fig. 13: das Implantat mit Führungskatheter mit Stopper
- Fig. 14a und b: den Stopper am Führungskatheter

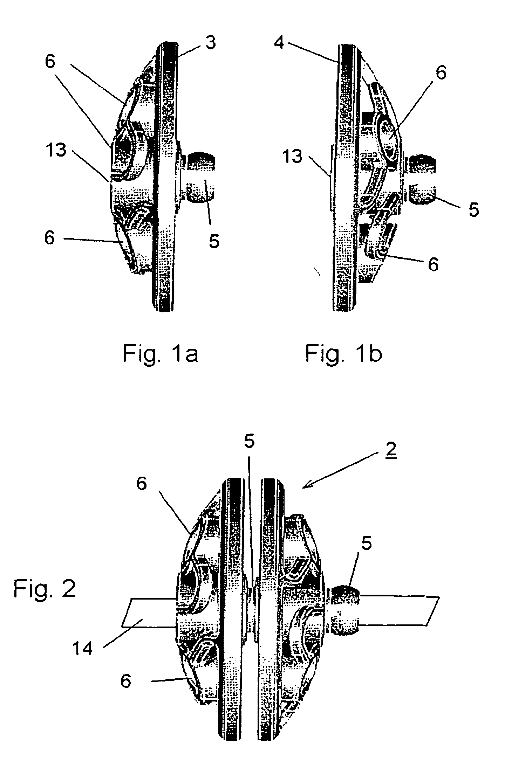

In den Figuren 1a und 1b sind zwei nach der Erfindung mögliche Modulteile 3 und 4 dargestellt. Die beiden Modulteile 3 und 4 sind im Umfang kalottenförmig ausgebildet, wobei im Zentrum der Modulteile 3 und 4 jeweils Steckverbindungen 5 angeordnet sind. Die Steckverbindungen können einstückig an den Modulteilen 3 und 4 angebracht sein. Ebenso sind separate Clips geeignet, die Modulteile steckbar zu einem Modulpaar 2 zusammenzufügen, wie es in Figur 2 gezeigt ist. Das Modulpaar 2 bildet die kleinstmögliche Ausführungsform eines erfindungsgemässen Implantates 1. In den Modulteilen 3 und 4 sind Durchführungen 6 für Katheter oder Führungselemente für radiologische Quellen zur Behandlung der kranken Bereiche im Resektionshohlraum angeordnet. Der beispielsweise durch das Zentrum 13 geführte Katheter 14 ist nach der Behandlung heraus ziehbar, wobei er sich von den Modulpaaren (2, 9, 10, 11) löst.

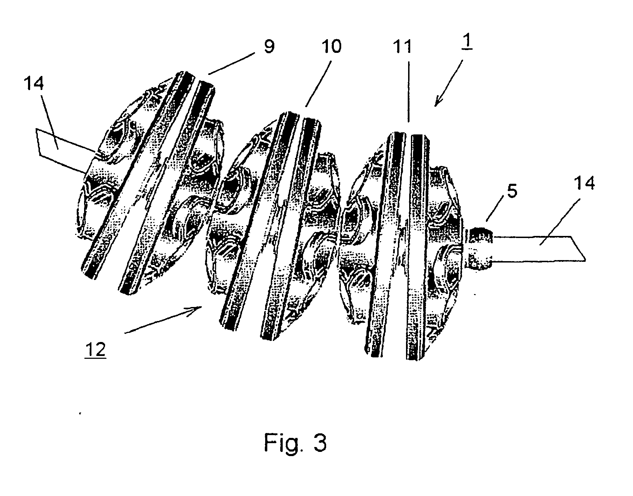

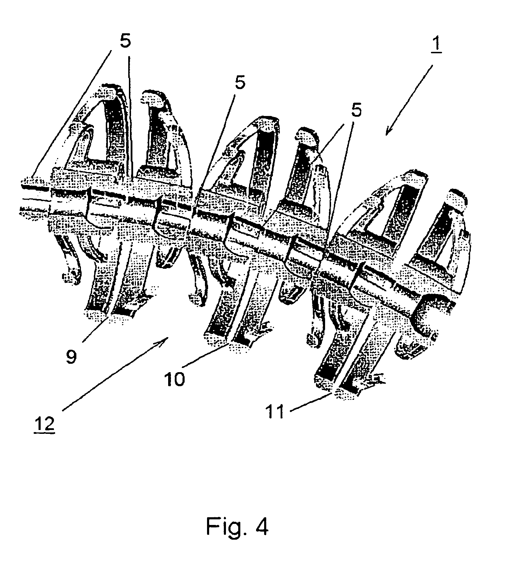

Die Figur 3 zeigt ein Implantat 1, welches aus drei Modulpaaren (9, 10, 11) aufgebaut ist und eine Kette 12 bildet. Die Modulpaare (9, 10, 11) bzw. die Kette 12 sind aus biologisch abbaubarem Material und bleiben nach der Behandlung in dem Hohlraum. Um eine radiologische Erkennung zu ermöglichen sind dem Material Stoffe beigefügt, die im Röntgenbild sichtbar werden. In Figur 4 ist das Implantat von innen zu sehen.

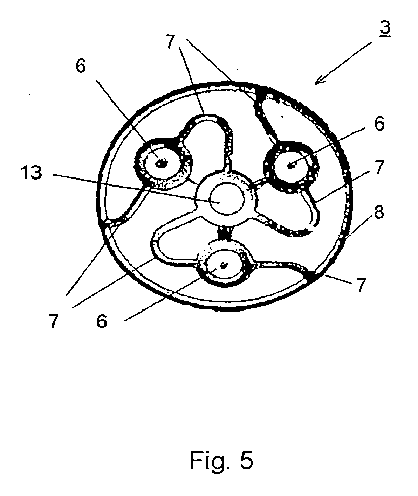

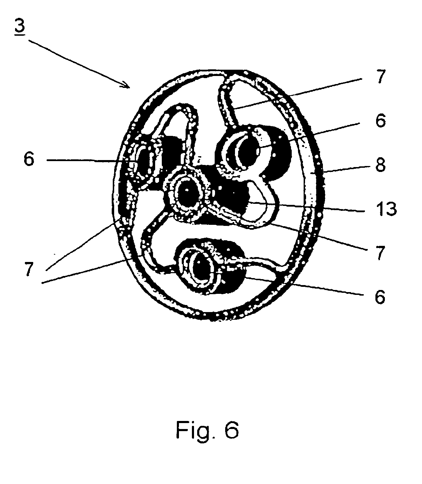

Den inneren Aufbau eines Modulteiles 3 zeigen die Figuren 5 und 6. Innerhalb des Umfangs 8 des Modulteiles 3 sind in diesem Beispiel vier Durchführungen 6 für Katheter bzw. Führungselemente für Behandlungseinrichtungen, beispielsweise radiologische Quellen zur Strahlungsbehandlung, angeordnet. Neben der zentralen Durchführung im Zentrum 13 des Modulteiles 3 sind drei exzentrische Durchführungen 6 vorgesehen. Dies ermöglicht eine nähere Platzierung der Behandlungseinrichtung zum Erkrankungsherd. Die einzelnen Durchführungen 6 sind mittels federnden Elementen 7 untereinander, mit dem Zentrum 13 und dem Umfang 8 elastisch zur Gewährung einer optimalen Flexibilität (nicht zu hoch und nicht zu steif) verbunden. In diesem Ausführungsbeispiel sind die federnden Elemente 7 S-förmig ausgebildet. Ebenso sind alle denkbaren federnden Formgebungen denkbar. Der Aussenring am Umfang 8 kann durchgehend oder unterbrochen sein.

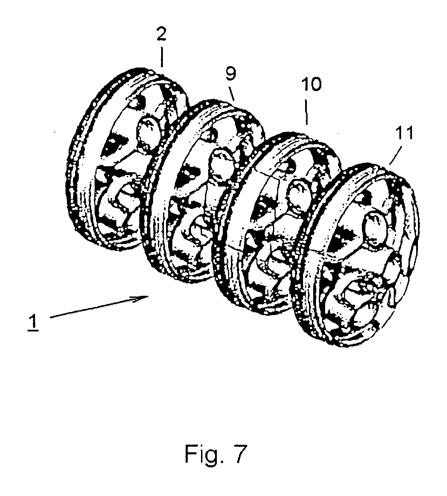

Die Figur 7 zeigt ein kettenförmiges Implantat 1, bestehend aus vier Modulpaaren 2, 9, 10, 11, 12. Die Anzahl der Modulpaare ist gemäss der Erfindung nicht begrenzt. Je nach Anwendung kann die Kette beliebig viele Modulpaare aufweisen.

Die mit der Erfindung verbundene Vorteile bestehen insbesondere darin, dass durch den modularen Aufbau des Implantats für viele Anwendungen einsetzbar ist und eine hohe Flexibilität sowohl in Längsrichtung als auch in radialer Richtung in den Modulteilen aufweist. Darüber hinaus kann die Behandlung der Wände des Resektionshohlraumes genügend nahe zum behandelnden Bereich erfolgen.

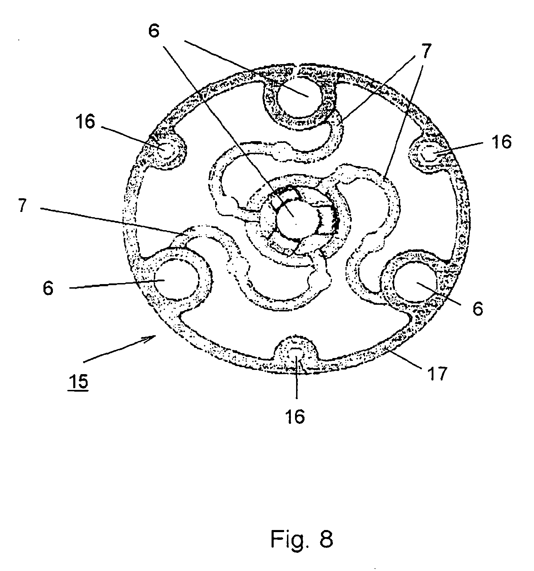

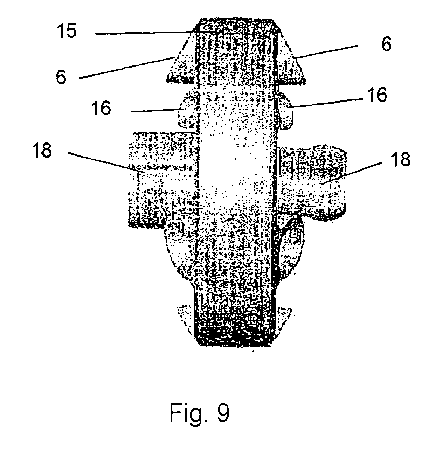

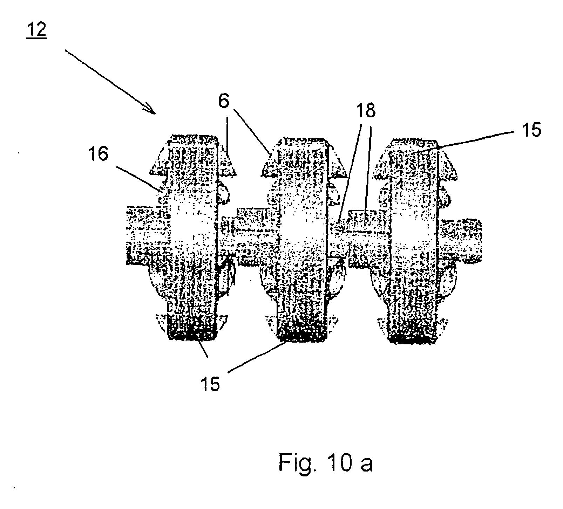

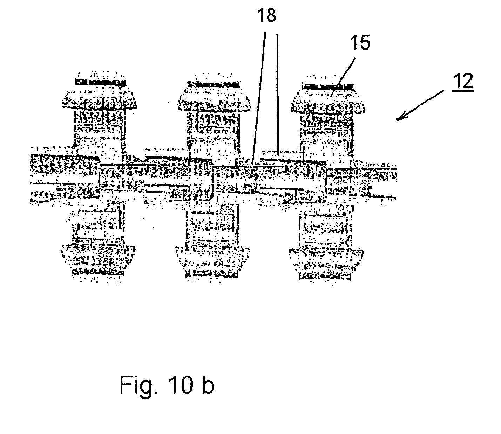

Die Figuren 8 bis 10 zeigen ein weiteres Ausführungsbeispiel eines Implantates 1. Dieses Implantat 1 besteht aus mindestens einem Modul 15, welches einstückig ausgebildet ist und wie die Modulpaare in dem vorherigen Ausführungsbeispiel Durchführungen 6 für die Katheter bzw. radiologischen Quellen zur Therapie aufweist. Ferner sind federnde Elemente 7 vorgesehen. Die federnden Elemente 7 können alle denkbaren Formen aufweisen, wie zum Beispiel bogenförmig, S-förmig, zickzackförmig, C-förmig oder mittels kleiner Federn etc. Diese in radialer Richtung federnden Elemente können aus flexiblem Kunststoff, beispielsweise Polymeren bestehen. Wesentlich bei den federnden Elementen 7 ist die elastische Lagerung der mittleren Durchführung 6 für den Führungskatheter. Im Inneren des Moduls 15 sind am Umfang des Aussenrings 17 des Moduls 15 kleine Durchführungen 16 ausgebildet, die zur Einführung eines Stoffes, der radiologisch erkennbar ist, dient. Beispielsweise kann der Stoff aus Magnesiumlegierungen bestehen. Die Seitenansicht in Figur 9 zeigt das Modul 15 mit den Durchführungen 6. Die Durchführungen 6 sind so geformt, dass die Einhüllende der Modulseiten beidseitig Kreisabschnitte bildet. An den Modulen 15 sind beidseitig einstückig Steckverbindungen 18 angebracht. Die Steckverbindungen sind in der Weise ausgestaltet, dass die einzelnen Module miteinander zu einer Kettenbildung verbindbar sind, wie es in den Figuren 10a und 10b gezeigt ist. Die Figur 10 a zeigt die Kette 12 geschlossen in linearer Form, die Figur 10b in aufgeschnittener Darstellung.

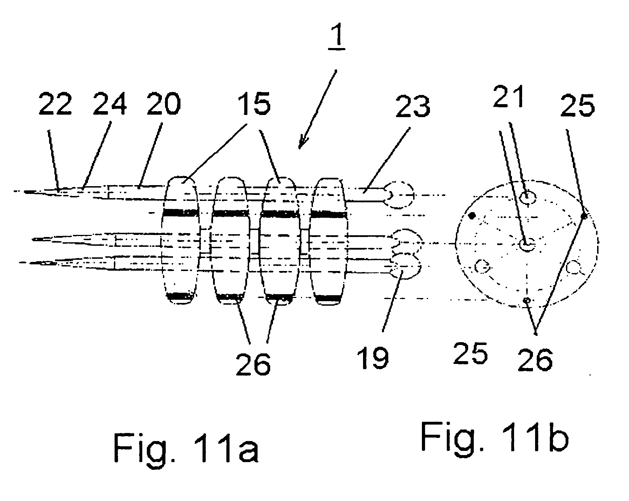

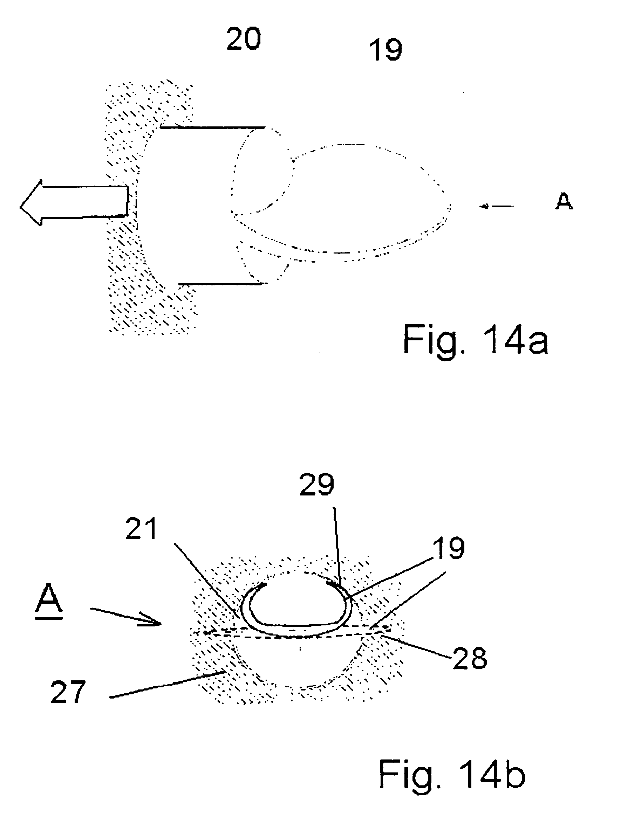

Ein erfindungsgemässes Implantatsystem 1 ist in den Figuren 11a (Seitenansicht) und 11b (Querschnitt) gezeigt. In Figur 11a sind vier Module 15 mit drei Führungskathetern 20 zu sehen, welche durch die Durchführungen 21 geführt sind. Die Führungskatheter 20 weisen am distalen Ende 22 Nadeln 24 und am proximalen Ende 23 Stopper 19 auf. Die Führungskatheter 20 bestehen aus biologisch abbaubarem und antiseptischem Material. In den Öffnungen 25 können beispielsweise Magnesiumstifte 26 zur Sichtbarmachung des Implantates auf einem CT-Monitor angeordnet sein. Die Module 15 sind mit Steckverbindungen 5 (wie oben beschrieben) torsionsgesichert verbunden. Das Implantat 1 bleibt im resektionierten Hohlraum so positioniert, dass eine Relativbewegung zwischen System und Körper verhindert wird. Die Führungskatheter 20 ist einseitig verschlossen. Der am proximalen Ende angeordnete Stopper 19 fixiert den Führungskatheter 20 nach der Implantation und verhindert Relativbewegungen. Der Führungskatheter 20 ist unter Zug (beispielsweise F > 6 Newton) aus dem Körper entfernbar, wobei der Stopper 19 geeignet (siehe Figuren 14a und 14b) verformbar ist.

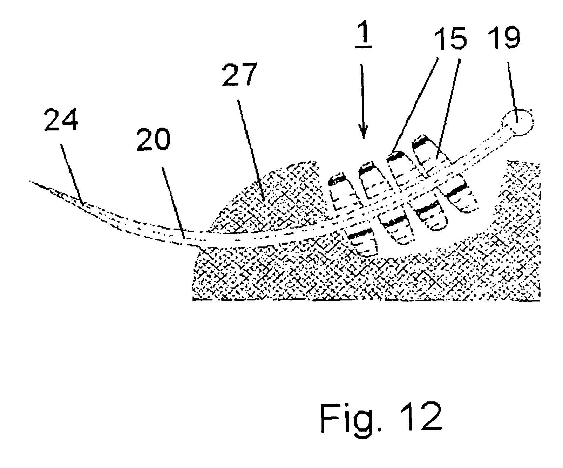

In Figur 12 ist die Einführung des Implantates 1 in den Körper zu sehen. Der Führungskatheter 20 ist axial zu den Modulen 15 bis zum Stopper 19 verschiebbar. Die Nadel 24 dient zum Durchstossen des Gewebes 27. Sie wird nach der Implantation vom Führungskatheter 20 getrennt.

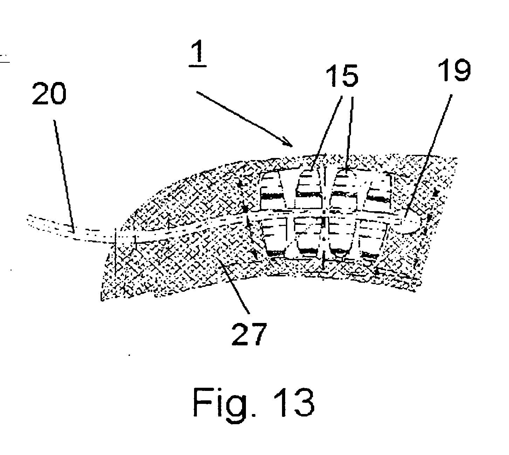

Das im Körper implantierte System 1 zeigt die Figur 13. Das Implantat 1 ist in seinem Schwerpunkt relativ zu dem behandelten Bereich fixiert. Bei Bewegungen des Körperteils bewegt sich das Implantat 1 mit. Der Stopper 19 am geschlossenen, proximalen Ende 23 fixiert den Führungskatheter 20. Bei Formveränderungen des volumenkonstanten Körperteils durch Bewegungen kann sich das Körperteil relativ zum Führungskatheter 20 bewegen, ohne dass der Führungskatheter 20 deformiert wird.

Die Figuren 14a und 14b zeigen den Stopper 19 und seine Funktion. Der Stopper 19 des Führungskatheters 20 ist unter Zug plastisch verformbar. Die hierfür erforderliche Zugkraft liegt in der Grössenordnung von 3 bis 10 Newton. Die plastische Deformation passt sich infolge der Zugkraft dem freien Durchlass der Durchführung 21 an, wie es in Figur 14b zu sehen ist. Die gestrichelte Linie 28 zeigt den Stopper 19 in Stoppfunktion, die durchgezogene Linie 29 den deformierten Stopper 19, wie er durch die Durchführung 21 geführt wird. Die plastische Verformung verhindert Sprödbruch und damit nicht gewünschte Partikelrückstände im Körper.

### Bezugszeichenliste

- 1: Implantat
- 2: Modulpaar
- 3: Modulteil
- 4: Modulteil
- 5: Steckverbindung
- 6: Durchführung
- 7: Federnde Elemente
- 8: Umfang
- 9: Modulpaar
- 10: Modulpaar
- 11: Modulpaar
- 12: Kette
- 13: Zentrum
- 14: Katheter
- 15: Modul
- 16: Durchführungen
- 17: Aussenring
- 18: Steckverbindung
- 19: Stopper
- 20: Führungskatheter
- 21: Durchführung
- 22: Distales Ende
- 23: Proximales Ende
- 24: Nadel
- 25: Öffnung
- 26: Magnesiumstifte
- 27: Gewebe
- 28: Gestrichelte Linie
- 29: Durchgezogene Linie

## Patentansprüche

1. Implantat (1) zur Behandlung von einem Hohlraum infolge einer Resektion, mit eine Kette bildenden Modulpaaren (2, 9, 10, 11) oder Modulen (15), wobei ein Modulpaar (2, 9, 10, 11) durch zwei Modulteile (3, 4) gebildet ist, wobei das Implantat einen mit einem Stopper (19) versehenen Führungskatheter (20) aufweist, wobei der Führungskatheter (20) durch eine in den Modulpaaren (2, 9, 10, 11) oder Modulen (15) angeordnete Durchführung (6,16) ein- und ausführbar ist, **dadurch gekennzeichnet, dass** die Modulteile (3, 4) der Modulpaare (2, 9, 10, 11) oder die Module (15) federnde Elemente (7) aufweisen, wobei die federnden Elemente (7) mit dem Umfang (8) und den Durchführungen (6) der Modulteile (3, 4) der Modulpaare (2, 9, 10, 11) oder der Module (15) verbunden sind.

2. Implantat nach Anspruch 1 **dadurch gekennzeichnet, dass** ein Modul (15) im Umfang beidseitig kalottenförmig und einstückig ausgebildet ist.

3. Implantat nach mindestens einem der Ansprüche 1 oder 2, **dadurch gekennzeichnet, dass** die Modulpaare (2, 9, 10, 11), Modulteile (3, 4) oder Module (15) hohle Steckeinrichtungen (5) aufweisen, mit denen die Modulteile (3, 4), Modulpaare (2, 9, 10, 11) oder Module (15) miteinander verbindbar sind.

4. Implantat nach mindestens einem der Ansprüche 1 oder 2, **dadurch gekennzeichnet, dass** die Modulpaare (2, 9, 10, 11), Modulteile (3, 4) oder Module (15) auf einem Führungskatheter (20) lose aufgereiht sind.

5. Implantat nach mindestens einem der vorherigen Ansprüche, **dadurch gekennzeichnet, dass** der Führungskatheter (20) durch die hohlen Steckeinrichtungen (5) ein- und ausziehbar sind.

6. Implantat nach mindestens einem der vorherigen Ansprüche, **dadurch gekennzeichnet, dass** die Steckverbindungen (5) als Muffen- Steckverbindung ausgebildet sind.

7. Implantat nach mindestens einem der vorherigen Ansprüche, **dadurch gekennzeichnet, dass** die Steckverbindungen (5) gegen Torsion gesichert sind.

8. Implantat nach mindestens einem der vorherigen Ansprüche, **dadurch gekennzeichnet, dass** die Modulteile (3, 4) oder Module (15) Durchführungen (6) für radiologische Quellen und/oder Katheter aufweisen.

9. Implantat nach Anspruch 1, **dadurch gekennzeichnet, dass** die federnden Elemente (7) S-, Z-, schraubenfederförmig sind oder aus elastischem Material bestehen, um die notwendige Flexibilität des Implantats (1) und/oder Module (15) zu erhalten.

10. Implantat nach Anspruch 1 oder 9, **dadurch gekennzeichnet, dass** die federnden Elemente (7) wie Speichen zwischen dem Aussenring (17) und/oder Aussendurchführungen (16) und der zentralen Durchführungen (6) angeordnet sind.

11. Implantat nach mindestens einem der vorherigen Ansprüche, **dadurch gekennzeichnet, dass** das Implantat (1) aus mindestens einem Modulpaar (2, 9, 10, 11) oder einem Modul (15) besteht, wobei die Modulpaare (2, 9, 10, 11) oder Module (15) eine flexible Kette (12) bilden.

12. Implantat nach mindestens einem der vorherigen Ansprüche, **dadurch gekennzeichnet, dass** der Führungskatheter (20) an einem Ende (22) eine Nadel (24) und am anderen Ende (23) einen Stopper (19) aufweist.

13. Implantat nach Anspruch 12, **dadurch gekennzeichnet, dass** die Nadel (24) entfernbar ist.

14. Implantat nach Anspruch 12, **dadurch gekennzeichnet, dass** der Stopper (19) verformbar ist.

15. Implantat nach mindestens einem der vorherigen Ansprüche, **dadurch gekennzeichnet, dass** das Implantat (1) aus einem biologisch abbaubaren Material besteht.

16. Implantat nach Anspruch 12, **dadurch gekennzeichnet, dass** der Führungskatheter (20) mit einem antiseptischen Material, welches im Röntgenbild sichtbar ist, imprägniert ist.

17. Implantat nach Anspruch 12 und/oder 16, **dadurch gekennzeichnet, dass** das Führungsrohr des Führungskatheter (20) in den Innenwänden oder im inneren des Katheterhohlraumes für Röntgenstrahlen sichtbare Drähte aufweist.

18. Implantat nach mindestens einem der vorherigen Ansprüche, **dadurch gekennzeichnet, dass** das Implantat (1) aus einem biologisch abbaubaren Material ist, wobei das Material für die Anwendung geeignet elastisch ist.

19. Implantat zur Behandlung von einem Hohlraum infolge einer Resektion nach Anspruch 1, **dadurch gekennzeichnet, dass** die Modulteile (3, 4), die Modulpaare (2, 9, 10) oder die Module (15) Durchführungen (16) zur Zuführung oder Anordnung von röntgenologisch oder im CT-Verfahren erkennbaren und biologisch abbaubaren Indikatoren (26) aufweisen.

## Claims

1. Implant (1) for treating a cavity as a result of a resection, with module pairs (2, 9, 10, 11) or modules (15) forming a chain, whereby a module pair (2, 9, 10, 11) is formed by two module parts (3, 4), whereby the implant has a guide catheter (20) provided with a stopper (19), whereby the guide catheter (20) can be introduced and withdrawn through a duct (6, 16) arranged in the module pairs (2, 9, 10, 11) or modules (15), **characterized in that** the module parts (3, 4) of the module pairs (2, 9, 10, 11) or the modules (15) have resilient elements (7), whereby the resilient elements (7) are connected to the circumference (8) and the ducts (6) of the module parts (3, 4) of the module pairs (2, 9, 10, 11) or the modules (15).

2. Implant according to Claim 1 **characterized in that** a module (15) is designed dome-shaped and one-piece in circumference on both sides.

3. Implant according to at least any one of Claims 1 or 2, **characterized in that** the module pairs (2, 9, 10, 11), module parts (3, 4) or modules (15) have hollow plug-in devices (5), by which the module parts (3, 4), module pairs (2, 9, 10, 11) or modules (15) can be connected to one another.

4. Implant according to at least any one of Claims 1 or 2, **characterized in that** the module pairs (2, 9, 10, 11), module parts (3, 4) or modules (15) can be strung loosely on a guide catheter (20).

5. Implant according to at least any one of the preceding claims, **characterized in that** the guide catheter (20) can be introduced and withdrawn through the hollow plug-in devices (5).

6. Implant according to at least any one of the preceding claims, **characterized in that** the plug-in connections (5) are designed as a sleeve plug-in connection.

7. Implant according to at least any one of the preceding claims, **characterized in that** the plug-in connections (5) are secured against torsion.

8. Implant according to at least any one of the preceding claims, **characterized in that** the module parts (3, 4) or modules (15) have ducts (6) for radiological sources and/or catheters.

9. Implant according to Claim 1, **characterized in that** the resilient elements (7) are shaped like an S, Z or helical spring or comprise elastic material to obtain the required flexibility of the implant (1) and/or modules (15).

10. Implant according to Claim 1 or 9, **characterized in that** the resilient elements (7) are arranged like spokes between the outer ring (17) and/or external ducts (16) and the central ducts (6).

11. Implant according to at least any one of the preceding claims, **characterized in that** the implant (1) comprises at least one module pair (2, 9, 10, 11) or a module (15), with the module pairs (2, 9, 10, 11) or modules (15) forming a flexible chain (12).

12. Implant according to at least any one of the preceding claims, **characterized in that** the guide catheter (20) has a needle (24) at one end (22) and a stopper (19) at the other end (23).

13. Implant according to Claim 12, **characterized in that** the needle (24) can be removed.

14. Implant according to Claim 12, **characterized in that** the stopper (19) can be deformed.

15. Implant according to at least any one of the preceding claims, **characterized in that** the implant (1) comprises a biodegradable material.

16. Implant according to Claim 12, **characterized in that** the guide catheter (20) is impregnated with an antiseptic material visible in the X-ray image.

17. Implant according to Claim 12 and/or 16, **characterized in that** the guide tube of the guide catheter (20) in the inner walls or inside the catheter cavity has wires visible for X-rays.

18. Implant according to at least any one of the preceding claims, **characterized in that** the implant (1) is made of a biodegradable material, whereby the material is appropriately elastic for use.

19. Implant for treating a cavity as a result of a resection according to Claim 1, **characterized in that** the module parts (3, 4), the module pairs (2, 9, 10) or the modules (15) have ducts (16) for supplying or arranging biodegradable indicators (26) which can be recognised radiologically or by the CT method.

## Revendications

1. Implant (1) pour le traitement d'une cavité suite à une résection, qui présente des paires (2, 9, 10, 11) de modules ou des modules (15) qui forment une chaîne, une paire (2, 9, 10, 11) de modules étant formée par deux parties de module (3, 4),
l'implant présentant un cathéter de guidage (20) doté d'un arrêt (19),
le cathéter de guidage (20) pouvant être inséré et extrait par un passage (6, 16) disposé dans les paires (2, 9, 10, 11) de modules ou dans le module (15),
**caractérisé en ce que**
les parties de module (3, 4) des paires (2, 9, 10, 11) de modules ou les modules (15) présentent des éléments élastiques (7) et
**en ce que** les éléments élastiques (7) sont reliés à la périphérie (8) et au passage (6) des parties de module (3, 4) des paires (2, 9, 10, 11) de modules ou des modules (15).

2. Implant selon la revendication 1, **caractérisé en ce qu'**un module (15) a une périphérie en calotte sur ses deux faces et est formé d'un seul tenant.

3. Implant selon au moins l'une des revendications 1 ou 2, **caractérisé en ce que** les paires (2, 9, 10, 11) de modules, les parties de module (3, 4) ou les modules (15) présentent des dispositifs creux d'enfichage (5) par lesquels les parties de module (3, 4), les paires (2, 9, 10, 11) de modules ou les modules (15) peuvent être reliés les uns aux autres.

4. Implant selon au moins l'une des revendications 1 ou 2, **caractérisé en ce que** les paires (2, 9, 10, 11) de modules, les parties de module (3, 4) ou les modules (15) sont rangés lâchement sur un cathéter de guidage (20).

5. Implant selon au moins l'une des revendications précédentes, **caractérisé en ce que** le cathéter de guidage (20) peut être inséré et extrait par les dispositifs creux d'enfichage (5).

6. Implant selon au moins l'une des revendications précédentes, **caractérisé en ce que** les raccords enfichables (5) sont configurés comme raccords à manchon et fiche.

7. Implant selon au moins l'une des revendications précédentes, **caractérisé en ce que** les raccords enfichables (5) sont bloqués en torsion.

8. Implant selon au moins l'une des revendications précédentes, **caractérisé en ce que** les parties de module (3, 4) ou les modules (15) présentent des passages (6) pour une source radiologique et/ou un cathéter.

9. Implant selon la revendication 1, **caractérisé en ce que** les éléments élastiques (7) sont en forme de S, de Z ou de vis élastiques ou sont constitués d'un matériau élastique pour obtenir la flexibilité nécessaire de l'implant (1) et/ou des modules (15).

10. Implant selon les revendications 1 ou 9, **caractérisé en ce que** les éléments élastiques (7) sont disposés à la manière de rayons entre l'anneau extérieur (17) et/ou les passages extérieurs (16) et les passages centraux (6).

11. Implant selon au moins l'une des revendications précédentes, **caractérisé en ce que** l'implant (1) est constitué d'au moins une paire (2, 9, 10, 11) de modules ou d'un module (15), les paires (2, 9, 10, 11) de modules ou les modules (15) formant une chaîne (12) flexible.

12. Implant selon au moins l'une des revendications précédentes, **caractérisé en ce que** le cathéter de guidage (20) présente une aiguille (24) à une extrémité (22) et un arrêt (19) à son autre extrémité (23).

13. Implant selon la revendication 12, **caractérisé en ce que** l'aiguille (24) peut être retirée.

14. Implant selon la revendication 12, **caractérisé en ce que** l'arrêt (19) est déformable.

15. Implant selon au moins l'une des revendications précédentes, **caractérisé en ce que** l'implant (1) est constitué d'un matériau biologiquement déformable.

16. Implant selon la revendication 12, **caractérisé en ce que** le cathéter de guidage (20) est imprégné d'un matériau antiseptique visible dans une image radiologique.

17. Implant selon les revendications 12 et/ou 16, **caractérisé en ce que** le tube de guidage du cathéter de guidage (20) présente des fils visibles aux rayons X dans ses parois intérieures ou à l'intérieur de la cavité du cathéter.

18. Implant selon au moins l'une des revendications précédentes, **caractérisé en ce que** l'implant (1) est constitué d'un matériau biodégradable, le matériau présentant une élasticité qui convient pour l'application.

19. Implant selon la revendication 1, pour le traitement d'une cavité suite à une résection, **caractérisé en ce que** les parties de module (3, 4), les paires (2, 9, 10) de modules ou les modules (15) présentent des passages (16) qui permettent d'amener ou d'installer des repères biodégradables (26) détectables par radiologie ou par un procédé à CT.
